Europäisches Patentamt

European Patent Office

Office européen des brevets

(11) Veröffentlichungsnummer: **0 199 254**
**B1**

(12) **EUROPÄISCHE PATENTSCHRIFT**

(45) Veröffentlichungstag der Patentschrift: **16.08.90**

(21) Anmeldenummer: **86105158.9**

(22) Anmeldetag: **15.04.86**

(51) Int. Cl.⁵: **G 01 N 33/94, G 01 N 33/533**

(54) **Gesamt-Digoxin-Bestimmung und hierfür geeignetes Reagentienkit.**

(30) Priorität: **22.04.85 US 726255**

(43) Veröffentlichungstag der Anmeldung:
**29.10.86 Patentblatt 86/44**

(45) Bekanntmachung des Hinweises auf die
Patenterteilung:
**16.08.90 Patentblatt 90/33**

(84) Benannte Vertragsstaaten:
**AT BE CH DE FR GB IT LI NL SE**

(56) Entgegenhaltungen:
**EP-A-0 108 403**
**EP-A-0 115 332**
**DE-A-3 205 506**
**DE-A-3 230 527**
**US-A-4 056 468**

**The Mecck Index (ed 10) 1983, 3148**

(73) Patentinhaber: **F. HOFFMANN-LA ROCHE AG**
**Postfach 3255**
**CH-4002 Basel (CH)**

(72) Erfinder: **Farrenkopf, Bruce C.**
**84 Clinton Avenue**
**Clifton, N.J. 07011 (US)**
Erfinder: **Kaufman, Richard A.**
**744 Joralemon Street**
**Belleville, N.J. 07109 (US)**

(74) Vertreter: **Lederer, Franz, Dr. et al**
**Van der Werth, Lederer & Riederer**
**Patentanwälte Lucile-Grahn-Strasse 22**
**D-8000 München 80 (DE)**

**Beschreibung**

Damit eine Bestimmungsmethode genau ist, und diese im Falle bei dem die Konzentration des Analyten anhand einer Standardkurve bestimmt wird, müssen sich Standard- und Patientenprobe in der Bestimmungsmethode identisch verhalten. Einer der kritischsten Faktoren zwischen dem Standard- und der Patientenprobe ist die Wiederauffindung des Analyten im Standard, welche identisch sein muss mit der Wiederauffindung des Analyten der Patientenprobe, ansonsten falsche Werte entstehen. Dies ist besonders dann der Fall, wenn die gesamte Quantität eines Analyten in einer Patientenprobe zu messen ist.

In Fluoreszenzpolarisationsbestimmungen von Analyten in Serum ist eine der unerwünschten Eigenschaften dieser Technik der hohe Fluoreszenzhintergrund des Serums. Dies wird besonders dann problematisch, wenn niedrige Werte des Analyten, wie Dixogen, zu messen sind. In diesem Fall kann die Bestimmung nicht direkt im Serum mit einem Fluoresceinmarkierten Digoxinmarker durchgeführt werden, da die Serumhintergrundfluoreszenz das Signal, das vom Fluoresceinmarkierten Digoxinmarker ausgestrahlt wird, bei weitem übersteigt.

Um diese hohe Hintergrundfluoreszenz des Serums zu vermeiden, wurden proteinfreie Filtrate unter Verwendung von Perchlorsäure oder Trichloressigsäure verwendet. Obwohl diese Methoden bezüglich der Herstellung von proteinfreien Filtraten, die relativ frei von Hintergrundfluoreszenz sind, wirksam sind, zeigen sie andere Nachteile. Im Falle von Digoxin erhält man eine relativ geringe Wiederauffindung im Ueberstand und darüberhinaus ist die Wiederauffindung abhängig von der Proteinkonzentration in der Probe. Im diesem Zusammenhang vergleiche Porter et al., Clin. Chem. 30, 1826—1829 (1984).

Die Verwendung der Ultrafiltration zur Herstellung proteinfreier Filtrate ist ebenfalls bekannt. In einem solchen System wird das Protein selektiv verteilt in eine Fraktion des Probenvolumens (Retentat) wogegen freier Ligand im wesentlichen ungehindert mit Lösungsmittel in das Ultrafiltrat passiert. Systeme zur Durchführung dieser Ultrafiltration sind kommerziell erhältlich. Eines dieser Systeme ist das Centifree™ Mikroverteilufgssytem welches durch die Amicon Corporation in Danvers, Massachusetts verkauft wird. Dieses System ist im wesentlichen eine in sich geschlossene Filtereinheit, deren Filtermembran von solcher Porosität ist, dass ungefähr 99,9% der Proteine im Serum durch die Zentrifugalfiltration abgehalten werden. Die Werbeliteratur dieser Filtereinheit besagt, dass dieses System zur Abtrennung "freier" Serumanalyten, vorzugsweise Drogen, von der gebundenen Proteinfraktionen verwendet werden kann. So kann im Abwesenheit eines Dissoziationsmittels für die freie Droge, welche an das Protein gebunden ist, nur die freie Droge im Serum durch die Filtereinheit passieren und so in einer Bestimmungsmethode gemessen werden.

Berichte in der Literatur besagen, dass ungefährt 20—30% von Digoxin in Serum an Serumproteine gebunden ist. Vergleiche hierzu Pribor et al., Drug Monitoring and Pharmokinetic Data, Pathotex Publishers, Park Forrest South, Ill., p. 57 (1980) Im Falle, dass $^3$H-Digoxin menschlichem Serum beigefügt wird, würde man erwarten, dass man ungefährt 70—80% des Labels im Filtrat wiederauffinden würde. Unter Verwendung von 10 normalen menschlichen Serumproben, zu welchen $^3$H-Digoxen zugefügt wurde, und welche anschliessend durch Ultrafilter zentrifugal filtriert wurden, wurden gefunden, dass 15—25% des Digoxins in diesen Seren im Filtrat nicht wiedergefunden werden konnten, was bedeutet, dass sie vermutlich proteingebunden waren.

Die vorliegenden Erfindung betrifft eine Bestimmungsmethode für die Messung von Totalserumdigoxin unter Verwendung einer Kombination von Zentrifugalultrafiltration und eines Dissoziationsmittels, welches das Digoxin, welches an die Serumproteine gebunden ist, abspalten soll. Es wurde eine grosse Zahl von Verbindungen gefunden, die allein oder in Kombination in der Lage sind, proteingebundenes Digoxin von den Serumproteinen abzuspalten. Beispiele von wirksamen Dissoziationsmitteln umfassen Chinidin, Vitamin E (α-Tocopherol), niedere Alkanole und $C_3$—$C_{26}$-Fettsäuren, bevorzugt langkettige gesättigte und ungesättigte Fettsäuren. Effektive Konzentrationsbereiche wurden gefunden im Bereich von ungefähr 2 mg bis 50 mg pro 500 µl Serum für die Fettsäuren und α-Tocopherol, im Bereich von 50 µg bis 500 µg für 500 µl Serum für Chinidin und im Bereich von 3 mg bis 100 mg für niedere (gesättgte oder ungesättigte) Alkanole ($C_2$—$C_7$) wie Isopropylalkohol.

Resultate die man in Experimenten erhalten hat unter Zugabe von $^3$-H-Digoxin zu normalem Humanserum gefolgt von der Zugabe eines Dissoziationsmittels und Zentrifugalfiltration sind in der nachfolgenden Tabelle 1 erhalten.

TABELLE 1

| Dissoziations mittel | mg zugegeben/ 500 ml serum | % ³H-Digoxin wiederauffindung im Filtrat |
|---|---|---|
| 1. H₂O | 10 | 81 |
| 2. Arachidonsäure | 10 | 104 |
| 3. Linolensäure | 10 | 96 |
| 4. Vitamin E | 20 | 91 |
| 5. Chinidin | (200 µg) | 87 |
| 6. Linolsäure | 10 | 103 |
| 7. Oelsäure | 10 | 91 |
| 8. Isopropanol | 25 | 93 |
| 9. Linolensäure (16 mg) und Isopropanol (9 mg) | 25 | 98 |

Beispiel

Die Verwendung eines Dissoziationsmittels und der Zentrifugalultrafiltration wird unter Verwendung einer Fluoreszenzpolarisationsbestimmung für Digoxin auf dem COBAS BIO exemplifiziert.

Reagenzien:

1. Antikörperreagenz—0,2 Mol Tris(hydroxymethyl)aminomethan/Liter, pH 8,0 enthaltend Kaninchenanti-Digoxinserum, 0,01% Rindergammaglobulin, 0,1% Natriumazid und Surfactans.

2. Tracer Reagenz—0,05 Mol Tris(hydroxymethyl)aminomethan/Liter, pH 8,0, enthaltend 0,01% Rindergammaglobulin, 0,1% Natriumazid, Surfactants, und Fluuresceindigoxin-konjugat.

3. Digoxindissoziationsmittel—67% (v/v) Linolensäure in Isopropanol.

Diese Reagenzien können um genügend Reagens für Mehrfachbestimmungen zu erhalten in Kit-Form mit einer Flasche pro Reagens hergestellt werden. Das Reagens-Kit kann erwünschtenfalls ein oder mehrere Mikroverteilungsultrafilter enthalten.

Die Bestimmung wird wie folgt durchgeführt:

a) 500 µl Probe (Serum, Plasma, Speichel, Urin) werden zu einem Amicon Centrifree Mikroverteilungssystemfilter gegeben (eine anisotrope, hydrophile YMT Ultrafiltrationsmembran).

b) 25 µl des Digoxindissoziationsmittels werden zugegeben und der Filtereinhalt wird am Vortex gemischt.

c) Nach Installieren eines Auffangbehälters am Ende des Filters wird das Reaktionsgemisch vorzugsweise in eine Zentrifuge mit fixiertem Winkelrotor (35—45°) gegeben und bei 2000×g während 20 Minuten zentrifugiert.

d) Nach der Zentrifugation werden der Filter und der Auffangbehälter von der Zentrifuge entfernt, und das Ultrafiltrat enthaltend das Dioxin wird nach einer Fluoreszenzpolarisationsbestimmungsmethode zur Bestimmung der Digoxinkonzentration gemessen.

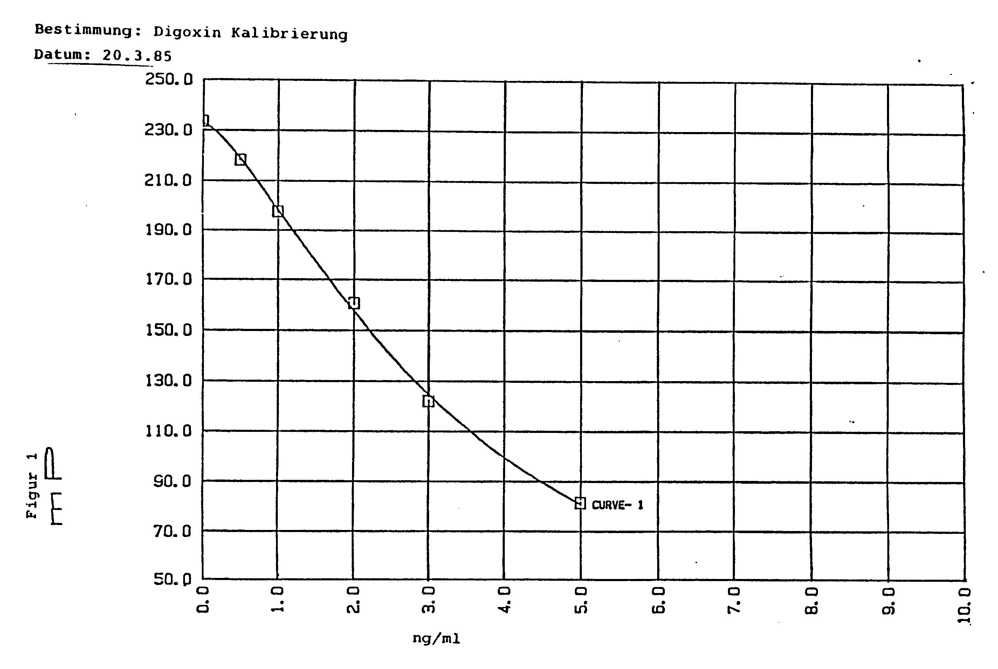

Ein Beispiel einer typischen Kalibrationskurve ist in Figur 1 gegeben.

**Patentansprüche**

1. Verfahren zur Bestimmung von Gesamtdigoxinmengen in einer flüssigen biologischen Probe durch Behandeln dieser Probe mit einer Lösung eines Dissoziationsmittels, ausgewählt aus einem oder mehreren Gliedern der Gruppe bestehend aus C₃—C₂₆ gesättigten oder ungesättigten Fettsäuren, eines C₂—C₇ niederen Alkanols, Chinidin und α-Tocopherol, um das an das Serumprotein gebundene Digoxin abzuspalten, Filtrieren der so behandelten Probe durch ein Zentrifugalultrafilter, so dass die das Digoxin enthaltende Probe selektiv dieses Filter passiert und die Serumproteine zurückbehalten werden, und anschliessendes Bestimmen des Digoxingehaltes in dieser Probelösung nach einer Fluoreszenzpolarisationsbestimmungsmethode.

2. Verfahren nach Anspruch 1, dadurch gekennzeichnet, dass das Dissoziationsmittel Linolensäure ist.

3

EP 0 199 254 B1

3. Verfahren nach Anspruch 1, dadurch gekennzeichnet, dass das Dissoziationsmittel Arachidonsäure ist.

4. Verfahren nach Anspruch 1, dadurch gekennzeichnet, dass das Dissoziationsmittel Linolsäure ist.

5. Verfahren nach Anspruch 1, dadurch gekennzeichnet, dass das Dissoziationsmittel Isopropylalkohol ist.

6. Verfahren nach Anspruch 1, dadurch gekennzeichnet, dass das Dissoziationsmittel ein Gemisch von Isopropylalkohol und Linolensäure ist.

7. Reagenzienkit zur Bestimmung von Totaldigoxinmengen nach Anspruch 1 in biologischen Flüssigkeiten, dadurch gekennzeichnet, dass das Kit in je einer Flasche die folgenden Reagenzien in genügender Menge zur Durchführung von Mehrfachbestimmungen enthält, nämlich.

(1). Anti-Digoxin-Antikörperreagenslösung;

(2). Tracerreagenslösung und

(3). Lösung eines Digoxindissoziationsmittels

8. Réagenzienkit gemäss Anspruch 7, dadurch gekennzeichnet, dass der Antidigoxin-antikörper ein Kaninchen-antidigoxinserum ist.

9. Reagenzienkit gemäss Anspruch 7, dadurch gekennzeichnet, dass das Digoxindissoziationsmittel ein Gemisch von Linolensäure in Isopropanol ist.

10. Reagenzienkit gemäss Anspruch 7, dadurch gekennzeichnet, dass die Tracerlösung ein Fluoresceindigoxinkonjugat ist.

11. Reagenzienkit gemäss Anspruch 7, dadurch gekennzeichnet, dass

(1). der Antidigoxin-antikörper ein Kaninchen-antidigoxinserum ist,

(2). das Digoxindissoziationsmittel ausgewählt aus der Gruppe bestehend aus Linolensäure, Arachidonsäure und Linolsäure ist und

(3). das Digoxintracerreagens eine Fluoresceindigoxinkonjugatlösung ist.

**Revendications**

1. Procédé pour doser les quantités totales de digoxine contenues dans un échantillon biologique liquide par traitement de cet échantillon par une solution d'un agent dissociant en un ou plusieurs représentants du groupe consistant en les acides gras saturés ou insaturés en C3—C26, les alcohols inférieurs en C2—C7, la quinidine et l'alpha-tocophérol, en vue de provoquer la scission de la digoxine fixée sur la protéine du sérum, filtration de l'échantillon ainsi traité sur un ultrafiltre centrifuge en sorte que l'échantillon contenant la digoxine passe sélectivement au travers de ce filtre et que les protéines du sérum soient retenues, puis dosage de la digoxine contenue dans cette solution-échantillon par une méthode de dosage par polarisation-fluorescence.

2. Procédé selon la revendication 1, caractérisé en ce que l'on utilise en tant qu'agent dissociant l'acide linolénique.

3. Procédé selon la revendication 1, caractérisé en ce que l'on utilise en tant qu'agent dissociant l'acide arachidonique.

4. Procédé selon la revendication 1, caractérisé en ce que l'on utilise en tant qu'agent dissociant l'acide· linoléique.

5. Procédé selon la revendication 1, caractérisé en ce que l'on utilise en tant qu'agent dissociant l'alcool isopropylique.

6. Procédé selon la revendication 1, caractérisé en ce que l'on utilise en tant qu'agent dissociant un mélange d'alcool isopropylique et d'acide linolénique.

7. Nécessaire de réactifs pour le dosage des quantités totales de digoxine dans des liquides biologiques selon la revendication 1, caractérisé en ce que le nécessaire contient, chacun dans un flacon, les réactifs suivants en quantité suffisante pour l'exécution de dosages répétés, à savoir:

(1). une solution de réactif anticorps-antidigoxine;

(2). une solution de réactif traceur, et

(3). une solution d'un agent dissociant de la digoxine.

8. Nécessaire de réactifs selon la revendication 1, caractérisé en ce que l'anticorps-anti-digoxine est un sérum anti-digoxine de lapins.

9. Nécessaire de réactifs selon la revendication 7, caractérisé en ce que l'agent dissociant de la digoxine est un mélange d'acide linolénique et d'isopropanol.

10. Nécessaire de réactifs selon la revendication 7, caractérisé en ce que la solution de traceur consiste en un produit de conjugaison fluorescéine-digoxine.

11. Nécessaire de réactifs selon la revendication 7, caractérisé en ce que:

(1). l'anticorps-anti-digoxine est un sérum anti-digoxine de lapins,

(2). l'agent dissociant de la digoxine est choisi dans le groupe consistant en l'acide linolénique, l'acide arachidonique et l'acide linoléique, et

(3). le réactif traceur de la digoxine est une solution d'un produit de conjugaison fluorescéine-digoxine.

4

**Claims**

1. A method for determining total digoxin levels in a liquid biological sample by treating this sample with a solution of a dissociation agent selected from one or more members of the group consisting of $C_3$—$C_{26}$ saturated or unsaturated fatty acids, a $C_2$—$C_7$ lower alkanol, quinidine and α-tocopherol in order to split off the digoxin bound to the serum protein, filtering the thus-treated sample through a centrifugal ultrafilter so that the sample containing the digoxin selectively passes this filter and the serum proteins are retained and subsequently determining the digoxin content in this sample solution according to a fluorescence polarization assay.

2. A method according to Claim 1, characterized in that the linolenic acid is the dissociation agent.

3. A method according to Claim 1, characterized in that the arachidonic acid is the dissociation agent.

4. A method according to Claim 1, characterized in that the linoleic acid is the dissociation agent.

5. A method according to Claim 1, characterized in that isopropyl alcohol is the dissociation agent.

6. A method according to Claim 1, characterized in that a mixture of isopropyl alcohol and linolenic acid is the dissociation agent.

7. A reagent kit for determining total digoxin levels in biological fluids according to Claim 1, characterized in that the kit comprises one bottle each of the following reagents in quantities sufficient to carry out multiple assays, namely

(1) anti-digoxin antibody reagent solution;

(2). tracer reagent solution and

(3). solution of a digoxin dissociation agent.

8. A reagent kit according to Claim 7, characterized in that the anti-digoxin antibody is a rabbit anti-digoxin serum.

9. A reagent kit according to Claim 7, characterized in that the digoxin dissociation agent is a mixture of linolenic acid in isopropanol.

10. A reagent kit according to Claim 7, characterized in that the tracer solution is a fluorescein digoxin conjugate.

11. A reagent kit according to Claim 7, characterized in that

(1). the anti-digoxin antibody is a rabbit anti-digoxin serum,

(2). the digoxin dissociation agent is selected from the group consisting of linolenic acid, arachidonic acid and linoleic acid and

(3) the digoxin tracer reagent is a fluorescein digoxin conjugate solution.

Figur 1

Bestimmung: Digoxin Kalibrierung
Datum: 20.3.85

ng/ml

CURVE-1